# EUROPÄISCHE PATENTSCHRIFT

(11) **EP 2 305 031 B1**
(45) Veröffentlichungstag und Bekanntmachung des Hinweises auf die Patenterteilung: **22.08.2012**
(21) Anmeldenummer: 10180670.1
(22) Anmeldetag: 29.05.1999
(51) Int. Cl.: A01N 51/00, B27K 3/50

(54) **Mittel zur Bekämpfung von Pflanzenschädlingen**
Agents for combatting plant pests
Agents pour lutter contre des parasites de végétaux

(30) Priorität: 10.06.1998 DE 19825891; 30.06.1998 DE 19829113
(43) Veröffentlichungstag der Anmeldung: 06.04.2011
(62) Teilanmeldung aus: 05016734.5
(73) Patentinhaber: Bayer CropScience AG, 40789 Monheim (DE)
(72) Erfinder: Erdelen, Christoph, 42799 Leichlingen (DE); Andersch, Wolfram, 51469 Bergisch Gladbach (DE); Stenzel, Klaus, 40595 Düsseldorf (DE); Mauler-Machnik, Astrid, 42799 Leichlingen (DE); Krämer, Wolfgang, 51399 Burscheid (DE)

(56) Entgegenhaltungen:
- WO-A-96/03045

## Beschreibung

Die vorliegende Erfindung betrifft Schädlingsbekämpfungsmittel die eine Wirkstoff. kombination der Verbindung der Formel (I) 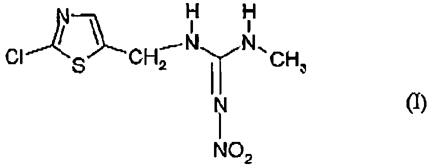 mit Thiran enthalten.

Die Verbindung der Formel (I) ist aus EP-OS 0 375 907 bekannt.

Fungizide Wirkstoffe, wie Azol-Derivate, Arylbenzylether, Benzamide, Morpholin-Verbindungen und andere Heterocyclen sind bekannt (vgl. K.H. Büchel "Pflanzenschutz und Schädlingsbekämpfung", Seiten 140 bis 153, Georg Thieme-Verlag, Stuttgart 1977, EP-OS 0 040 345, DE-OS 2 324 010, DE-OS 2 201 063, EP-OS 0 112 284, EP-OS O 304 758 und DD-PS 140 412).

Mischungen bestimmter Nitromethylenderivate mit fungiziden Wirkstoffen und ihre Verwendung als Schädlingsbekämpfungsmittel im Pflanzenschutz sind bereits bekannt (US-P-4 731 385; JP-OS 63-68507, 63/68505; 63/72 608; 63/72 609, 63/72 610, WO 96/03 045, JP 08 245 323, JP 04 368 303, JP 05 017 311, WO 97/22 254, WO 92/21 241). Mischungen bestimmter offenkettiger Nitromethylene und Nitroguanidine mit Fungiziden sind bereits bekannt (JP-OS 30 47 106; US-P 5 181 587).

Mischungen von Cyclopropylcarboxamiden mit bestimmten Nitromethylenen- oder Nitroguanidinderivaten sind bereits bekannt (JP-OS 3 271207).

Mischungen von u.a. Imidacloprid und fungiziden Wirkstoffen zur Anwendung im Materialschutz und gegen Termiten, nicht aber zur Anwendung gegen pflanzenschädigende Schädlinge sind bereits bekannt (EP-OS 0 511 541). Mischungen von Imidacloprid und Azolylmethylcycloalkanen insbesondere Triticonazol sind bekannt aus EP-OS 545 834.

Es ist jedoch noch nichts darüber bekannt geworden, daß sich Nitroguanidinderivate und Fungizide mit Ausnahme von Cyclopropylcarboxamiden und Triticonazol in ihrer Wirkung gegenseitig so günstig beeinflussen, daß sie bei guter Pflanzenverträglichkeit in hervorragender Weise als Bekämpfungsmittel gegen Pflanzenschädlinge eingesetzt werden können.

Die vorliegende Erfindung betrifft Mittel gegen Pflanzenschädlinge, die die Verbindung der Formel (I) 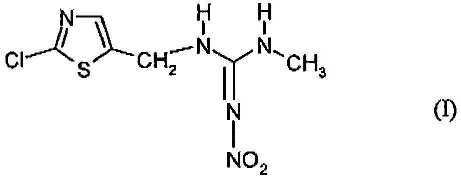 in Mischung mit
(33) einer Verbindung der Formel

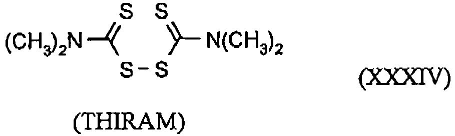

Die Verbindung ist beispielsweise beschrieben in K.H. Büchel, "Pflanzenschutz und Schädlingsbekämpfung, Seiten 121-153, Georg Thieme-Verlag, Stuttgart, 1977.

Die erfindungsgemäße Wirkstoffkombination Kann auch weitere Wirkstoffe sowie übliche Hilfs- und Zusatzstoffe sowie Verdünnungsmittel enthalten.

Wenn die Wirkstoffe in der erfindungsgemäßen Wirkstoffkombination in bestimmten Gewichtsverhältnissen vorhanden sind, zeigt sich ein deutlicher synergistischer Effekt der Mischungen. Jedoch können die Gewichtsverhältnisse der Wirkstoffe in der Wirkstoffkombination in einem relativ großen Bereich variiert werden. Im allgemeinen entfallen auf 1 Gewichtsteil an Wirkstoff der Formel (I)
0,1 bis 10 Gewichtsteile, vorzugsweise
0,3 bis 3 Gewichtsteile an einem fungiziden Wirkstoff (33).

Die erfindungsgemäße Wirkstoffkombination besitzt sehr gute fungizide Eigenschaften. Sie läßt sich vor allem zur Bekämpfung von phytopathogenen Pilzen, wie Plasmodiophoromycetes, Oomycetes, Chytridiomycetes, Zygomycetes, Ascomycetes, Basidiomycetes, Deuteromycetes usw. einsetzen.

Die erfindungsgemäße Wirkstoffkombination eignet sich besonders gut zur Bekämpfung von Getreidekrankheiten, wie Erysiphe, Cochliobolus, Septoria, Pyrenophora und Leptosphaeria, und gegen Pilzbefall an Gemüse, Wein und Obst, beispielsweise gegen Venturia oder Podosphaera an Äpfeln, Uncinula an Reben oder Sphaeroteca an Gurken.

Die Wirkstoffkombination eignet sich auch sehr gut zur Bekämpfung von tierischen Schädlingen, vorzugsweise Arthropoden, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus earpophagus, Scutigera spec.
Aus der Ordnung der Symphyla z.B, Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cyptomyzus ribis, Doralis fabae, Doralis pomi, Eriosoma lanigerum, Hyalopterus arundinis, Macrosiphum avenae, Myzus spp., Phorodon humuli, Rhopalosiphum padi, Phylloxera vastatrix, Pemphigus spp., Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Die gute Pflanzenverträglichkeit der Wirkstoffkombination in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäße Wirkstoffkombination kann in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithin, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäße Wirkstoffkombination kann in den Formulierungen in Mischung mit anderen bekannten Wirkstoffen vorliegen, wie Fungizide, Insektizide, Akarizide und Herbizide, sowie in Mischungen mit Düngemitteln oder Pflanzenwachstumsregulatoren.

Die Wirkstoffkombination kann als solche, in Form ihrer Formulierungen oder den daraus bereiteten Anwendungsformen, wie gebrauchsfertige Lösungen, emulgierbare Konzentrate, Emulsionen, Suspensionen, Spritzpulver, lösliche Pulver und Granulate, angewendet werden.

Die Anwendung geschieht in üblicher Weise, z.B. durch Gießen, Verspritzen, Versprühen, Verstreuen, Verstreichen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

## Patentansprüche

1. Mittel, enthaltend die Verbindung der Formel (I) 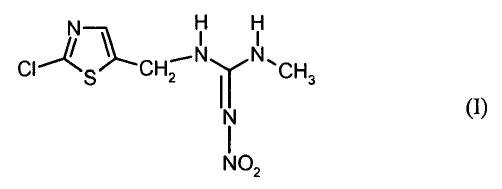 und
Thiram (33) der Formel (XXXIV)

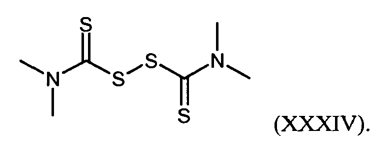

2. Mittel gemäß Anspruch 1, **dadurch gekennzeichnet, dass** in den Wirkstoffkombinationen auf 1 Gewichtsteil an Wirkstoff der Formel (I) 0,1 bis 10 Gew.-Teile an mindestens einem fungiziden Wirkstoff gemäß Thiram (33) der Formel (XXXIV) enfallen.

3. Verfahren zur Bekämpfung von Pilzen und Insekten, **dadurch gekennzeichnet, daß** man Wirkstoffkombinationen gemäß Anspruch 1 oder 2 auf die Pilze, Insekten und/oder deren Lebensraum einwirken läßt.

4. Verwendung von Wirkstoffkombinationen gemäß Anspruch 1 oder 2 zur Bekämpfung von Pilzen und Insekten.

5. Verfahren zur Herstellung von Mitteln gemäß Anspruch 1 oder 2, **dadurch gekennzeichnet, daß** man Wirkstoffkombinationen gemäß Anspruch 1 oder 2 mit Streckmitteln und/oder oberflächenaktiven Stoffen vermischt.

6. Saatgut, welches mit einer effektiven Menge des Mittels gemäß Anspruch 1 oder 2 behandelt ist.

7. Verwendung des Mittels gemäß Anspruch 1 oder 2 zur Behandlung von Saatgut.

## Claims

1. Composition containing the compound of the formula (I) 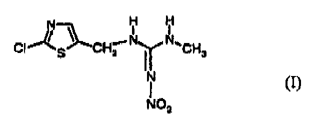 and
thiram (33) of the formula (XXXIV)

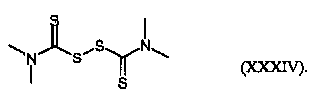

2. Composition according to Claim 1, **characterized in that** the active compound combinations contain from 0.1 to 10 parts by weight of at least one fungicidal active compound according to thiram (33) of the formula (XXXIV) per part by weight of active compound of the formula (1).

3. Process for controlling fungi and insects, **characterized in that** active compound combinations according to Claim 1 or 2 are allowed to act on the fungi, insects and/or their habitat.

4. Use of active compound combinations according to Claim 1 or 2 for controlling fungi and insects.

5. Process for preparing compositions according to Claim 1 or 2, **characterized in that** active compound combinations according to Claim 1 or 2 are mixed with extenders and/or surfactants.

6. Seed treated with an affective amount of the composition according to Claim 1 or 2.

7. Use of the composition according to Claim 1 or 2 for treating seed.

## Revendications

1. Composition contenant le composé de formule (I) 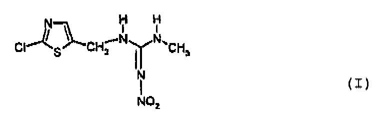 et
le thirame (33) de formule (XXXIV)

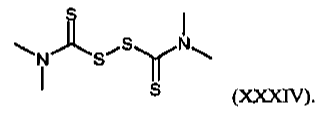

2. Composition selon la revendication 1, **caractérisée en ce que** dans l'association de substances actives pour 1 partie en poids de substances active de formule (I) sont présentes de 0,1 à 10 parties en poids d' au moins une substance active fongicide selon le thirame (33) de formule (XXXIV).

3. Procédé pour la lutte contre des champignons et des insectes, **caractérisé en ce qu'**on laisse agir les associations de substances actives selon la revendication 1 ou 2 sur les champignons, les insectes et/ou leur habitat.

4. Utilisation d' associations de substances actives selon la revendication 1 ou 2, pour la lutte contre des champignons et des insectes.

5. Procédé pour la préparation de compositions selon la revendication 1 ou 2, **caractérisé en ce qu'**on mélange des associations de substances actives selon la revendication 1 ou 2 avec des diluants et/ou des substances tensioactives.

6. Semence, qui a été traitée par une quantité efficace de la composition selon la revendication 1 ou 2.

7. Utilisation de la composition selon la revendication 1 ou 2, pour le traitement de semences.
